# EUROPÄISCHE PATENTSCHRIFT

(11) **EP 2 480 265 B1**
(45) Veröffentlichungstag und Bekanntmachung des Hinweises auf die Patenterteilung: **27.05.2015**
(21) Anmeldenummer: 10760581.8
(22) Anmeldetag: 17.09.2010
(51) Int. Cl.: A61M 1/06

(54) **BRUSTHAUBE ZUM ABPUMPEN VON MENSCHLICHER MUTTERMILCH**
BREAST SHIELD FOR EXPRESSING HUMAN BREAST MILK
TÉTERELLE POUR POMPER DU LAIT MATERNEL HUMAIN

(30) Priorität: 22.09.2009 US 244636 P
(43) Veröffentlichungstag der Anmeldung: 01.08.2012
(73) Patentinhaber: Medela Holding AG, 6340 Baar (CH)
(72) Erfinder: SCHLIENGER, André, CH-8933 Maschwanden (CH); WEBER, Beda, CH-5643 Sins (CH); FURRER, Etienne, CH-6300 Zug (CH); SILVER, Brian H., Cary IL 60013 (US); WÄCKERLIN, Daniela, CH-6340 Baar (CH); KHALIL, Gamal, CH-6340 Baar (CH)
(74) Vertreter: Clerc, Natalia
(86) Internationale Anmeldenummer: PCT/CH2010/000226
(87) Internationale Veröffentlichungsnummer: WO 2011/035448

(56) Entgegenhaltungen:
- WO-A1-2008/090386
- US-A1- 2004 087 898
- US-A1- 2005 222 536

## Beschreibung

### TECHNISCHES GEBIET

Die vorliegende Erfindung betrifft eine Brusthaube zum Abpumpen von menschlicher Muttermilch gemäss Oberbegriff des Patentanspruchs 1 sowie ein Brusthaubenset gemäss Oberbegriff des Patentanspruchs 11.

### STAND DER TECHNIK

Vorrichtungen zum Abpumpen von menschlicher Muttermilch sind hinlänglich bekannt. Grundsätzlich gibt es zwei verschiedene Typen: die ersten werden manuell bedient, d.h. der für das Abpumpen notwendige Unterdruck wird durch manuelle Bestätigung der Vakuumpumpe erzeugt. Bei den zweiten Typen ist die Vakuumpumpen elektrisch betrieben, wobei die Vakuumpumpe ans Stromversorgungsnetz angeschlossen sein kann und/oder über eine Batterie bzw. einen anderen Energiespeicher betrieben werden kann. Beispiele hierfür sind WO 96/22116, US 2009/0099511, US 2008/0287037, US 7 094 217 und US 2008/0039781.

Diese Vakuumpumpen sind entweder direkt oder über Vakuumleitungen mit einer Brusthaube verbunden. Die Brusthaube weist üblicherweise einen trichterförmigen Teil auf zur Aufnahme eines Teils der Mutterbrust inklusive der Brustwarze. In der Regel geht dieser trichterförmige Teil in einen hohlzylinderförmigen Teil über, an welchem einerseits entweder direkt die Vakuumpumpe oder die Saugleitung angeschlossen ist und welcher andererseits ebenfalls direkt oder über eine Milchleitung mit einem Milchsammelbehälter verbunden ist. Es ist bekannt, Brusthauben entsprechend der Grösse der Brust auszuwählen. Es sind insbesondere Brusthaubensets bekannt, bei welchen die Grösse des trichterförmigen Teils der Mutter entsprechend gewählt werden kann.

Es sind im Stand der Technik auch relativ kleine Brusthauben bekannt. So offenbart US 6 379 327 eine tragbare, sogenannte "hands-free" Abpumpvorrichtung. "hands-free" bedeutet in diesem Zusammenhang, dass die gesamte Vorrichtung nach dem Einschalten ohne Hände funktioniert, d.h. dass weder die Pumpe noch die Brusthaube von Hand gehalten werden müssen. Bei US'327 ist hierfür eine kleine trichterförmige Brusthaube mit Bändern an der Brust befestigt. Ein erster Schlauch führt von der Brusthaube zu einer Vakuumpumpe, welche in einem Gürtel gehalten ist. Ein zweiter Schlauch führt von der Brusthaube in einen Milchsammelbehälter, welcher im gleichen Gürtel getragen werden kann.

Auch WO 02/102437 und WO 2008/137678 zeigen "hands-free" Abpumpvorrichtungen. Hier ist die Brusthaube jeweils in einem Pumpengehäuse integriert und dient gleichzeitig als Membran zur Erzeugung eines Unterdrucks.

US 949 414 beschreibt eine trichterförmige Brusthaube, welche unter einem Büstenhalter angeordnet werden kann. Ein Vakuum wird nicht angelegt, sondern ein Schlauch führt von der Brusthaube zu einem Baby, welches durch Saugen am Schlauch die gewünschte Milch erhalten soll.

US 6 440 100 zeigt eine Vorrichtung zum Abpumpen von Muttermilch mit einer kleinen, unter dem Büstenhalter tragbaren Brusthaube. Ein Milchschlauch führt von der Brusthaube zu einem Milchsammelbehälter. Dieser ist über einen Vakuumschlauch mit einer Vakuumquelle verbunden. Der Milchsammelbehälter wird mittels der Vakuumquelle evakuiert, wobei der Unterdruck über den Milchschlauch an die Brusthaube angelegt wird. Dank dem im Milchsammelbehälter herrschenden Unterdruck soll nun abgepumpte Milch über den Milchschlauch in den Milchsammelbehälter gelangen. Alternativ kann der Milchsammelbehälter selber als Vakuumpumpe dienen. Diese Vorrichtung weist den Nachteil auf, dass das relativ grosse Volumen des Milchsammelbehälters ebenfalls evakuiert werden muss. Dieses sogenannte Totvolumen schränkt die Leistungsfähigkeit der Vorrichtung massiv ein.

US 2005/222536 offenbart eine Brusthaube in Form eines Hohlzylinders mit gerade verlaufendem Mantel. Die Brusthaube ist aus einem sehr weichen Material gefertigt.

US 2004/087898 zeigt eine Brusthaube mit einem Trichter und einer flexiblen Einlage. Diese Einlage kann sich in ihrer Form verändern, um die Brust zu massieren.

WO 2008/090386 betrifft eine Vorrichtung mit einer harten Schale und einem weichen Innenteil. Dieser Teil deformiert sich, wenn er auf eine Brust aufgesteckt wird. Der harte Teil kann Teil einer Brustpumpe sein, also eine Brusthaube, oder direkt vom Baby in den Mund genommen werden.

### DARSTELLUNG DER ERFINDUNG

Es ist eine Aufgabe der Erfindung, eine verbesserte Brusthaube zu schaffen, welche für die Mutter angenehm zu tragen ist und welche das luftgefüllte Totvolumen minimiert.

Diese Aufgabe lösen eine Brusthaube mit den Merkmalen des Anspruchs 1 sowie ein Brusthaubenset mit den Merkmalen des Anspruchs 11.

Die erfindungsgemässe Brusthaube weist einen rohrförmigen Stutzen und einen daran einstückig angeformten Trichter zur Auflage an eine Mutterbrust auf, wobei der Trichter sich zu seiner freien, dem Stutzen abgewandten Seite hin erweitert und wobei ein Kanal vorhanden ist, welcher sich von einem brustseitigen Ende des Trichters durchgehend bis zu einem, diesem Ende gegenüberliegenden, pumpenseitigen Ende des Stutzens erstreckt und welcher zum Anlegen eines Vakuums an die Mutterbrust und zum Abfliessen der abgepumpten Muttermilch dient. Erfindungsgemäss ist der Trichter flexibler ausgebildet als der Stutzen. Der Trichter weist einen sich über einen wesentlichen Teil seiner Länge erstreckenden Hauptbereich mit einem ersten Öffnungswinkel des Kanals und einen brustseitigen Endbereich mit einem zweiten Öffnungswinkel des Kanals auf. Der erste Öffnungswinkel ist bei Nichtgebrauch kleiner als der zweite Öffnungswinkel, wobei im Gebrauchszustand mindestens der erste Öffnungswinkel durch axialen Druck auf die Brusthaube vergrösserbar ist.

Diese Brusthaube passt sich optimal an die Form der menschlichen Mutterbrust an und schafft je nach Wunsch bzw. Anpressdruck eine dichte oder eine weniger dichte Verbindung mit der Brust.

Sie ist angenehm zu tragen und reduziert dank ihrer Anpassungsfähigkeit das luft- oder milchgefüllte Totvolumen.

Vorzugsweise ist die Brusthaube so klein ausgebildet, dass sie lediglich die Brustwarze und maximal die Areola umgibt. Diese lässt sich nicht nur einfach in einem "hands-free" System einsetzen, z.B. in einem Bürstenhalter halten, sondern sie weist aufgrund ihrer geringen Grösse kaum luftgefüllte Bereiche auf. Dies wirkt sich positiv auf die Vakuumpumpe aus, da diese weniger Leistung benötigt und somit geräuscharmer arbeiten kann. Des Weiteren lässt sie sich auch aus diesem Grund kleiner ausbilden.

Diese kleinen Brusthauben weisen zudem den Vorteil auf, dass weniger Gewebebewegung der Brust innerhalb der Haube stattfinden kann. Die Brusthauben können dadurch dichter am Gewebe anliegen. Dadurch ist wiederum eine geringere Pumpleistung notwendig. Die Pumpe kann ebenfalls kleiner ausgebildet werden und arbeitet geräuscharmer.

Im Gegensatz zu den bekannten Brusthauben und zum natürlichen Saugen eines Baby wird die Brustwarze in der erfindungsgemässen Brusthaube üblicherweise nicht auf das 2.5 fache ihrer Länge gedehnt. Dies ist für die Mutter, insbesondere bei schmerzenden Brustwarzen, angenehm.

Bei Nichtgebrauch sind typische Werte des ersten Öffnungswinkels der Brusthaube ≤ 5° (kleiner oder gleich 5°) und des zweiten Öffnungswinkels 90° bis 160°. Im Gebrauch und je nach angelegtem axialen Druck lässt sich zumindest der zweite Öffnungswinkel bis zu 120° oder vorzugsweise bis zu 160° vergrössern.

Vorzugsweise weist der Trichter einen brustseitigen Durchmesser von 5 mm bis 40 mm und eine Länge von 10 mm bis 40 mm auf, so dass im Gebrauchszustand die Brustwarze und maximal die Areola von der Brusthaube umfasst ist. Milchkanäle im Brustgewebe sind vorzugsweise nicht von der Brusthaube umgeben.

Vorzugsweise ist zwischen Stutzen und Trichter ein Übergangsbereich vorhanden mit einem dritten Öffnungswinkel des Kanals, wobei der dritte Öffnungswinkel bei Nichtgebrauch grösser ist als der erste Öffnungswinkel.

Typische Werte des dritten Öffnungswinkels sind 60° bis 150°.

Vorzugsweise schliesst der Hauptbereich unmittelbar an den Übergangsbereich an. Auch der Endbereich schliesst vorzugweise unmittelbar an den Hauptbereich an.

In einer bevorzugten Ausführungsform weist der Stutzen eine um ein Vielfaches grössere Wandstärke auf als der Trichter: Zusätzlich oder alternativ kann der Stutzen auch aus einem Material mit grösserer Shore Härte gebildet sein.

Vorzugsweise ist im Übergangsbereich zwischen Stutzen und Trichter ein äusserer Anschlag vorhanden, welcher dem äusseren Umfang des Stutzens vorsteht.

Der Stutzen lässt sich gut in eine Aufnahme einschieben, wenn er in seinem äusseren Umfang konisch zum Trichter hin erweiternd ausgebildet ist.

Diese erfindungsgemässe Brusthaube ist vorzugsweise aus Silikon gefertigt und weist vorzugsweise eine Shore A Härte von 30 bis 70 auf. Vorzugsweise weist der Trichter eine Shore A Härte von circa 50 und der Stutzen eine Shore A Härte von circa 70 auf.

Damit diese relativ kleine und kompakte Brusthaube gut in der Hand gehalten werden kann, ist sie vorzugsweise in einem Brusthaubenset mit einem Kopplungsteil angeboten. Das Kopplungsteil dient zur dichtenden Aufnahme des Stutzens der Brusthaube, wobei es zylinderförmig ausgebildet ist und auf einer Seite mit einem Boden verschlossen ist, so dass ein Sackloch zur Aufnahme des Stutzens gebildet ist. Es ist mindestens eine, vorzugsweise genau eine Anschlussöffnung vorhanden, welche in fluidkommunizierender Verbindung mit dem Kanal der Brusthaube steht.

Im zusammengebauten Zustand endet ein brusthaubenfernes, stirnseitiges Ende des Stutzens vorzugsweise beabstandet zum Boden des Kopplungsteils. Vorzugsweise ist die mindestens eine Anschlussöffnung azentrisch im Kopplungsteil angeordnet.

Ist die mindestens eine Anschlussöffnung in einem oberen Bereich des Kopplungsteils angeordnet und das Kopplungsteil weist eine Markierung auf, welche "oben" im Raum definiert, so werden beim Abpumpen stehende Luftblasen im Kopplungsteil vermieden und luftgefüllte Toträume eliminiert.

Um den konischen Stutzen einfacher einführen zu können, verjüngt sich auch das Sackloch des Kopplungsteils im Durchmesser zum Boden hin.

Vorzugsweise ist das Kopplungsteil steif ausgebildet, was die Einführung des Stutzens erleichtert und die Stabilität erhöht. Das Teil lässt sich somit einfacher halten oder befestigen.

Vorzugsweise weist die Brusthaube keinerlei Einsätze auf und besteht lediglich aus den oben genannten Elementen.

Die erfindungsgemässe Brusthaube lässt sich mit allen Arten von Brustpumpen kombinieren. Vorteilhaft ist jedoch ihre Verwendung mit einer Brustpumpe, welche während des Abpurnpens von einem pneumatischen in ein hydraulisches System wechselt. Das heisst, dass dieselbe Leitung, welche anfangs das Vakuum an die Brusthaube angelegt hat, auch zum Transport der abgepumpten Milch dient und somit die Milch das Medium wird, welches den weiteren Milchfluss aus der Mutterbrust erzeugt.

Eine derartige Vorrichtung zum Abpumpen von menschlicher Muttermilch weist die erfindungsgemässe Brusthaube zur Anlage an eine Mutterbrust, eine Vakuumpumpe zur Erzeugung eines Vakuums, eine die Brusthaube mit der Pumpkammer verbindende Leitung zum Übermitteln des erzeugten Vakuums an die Brusthaube und eine Kammer auf. Die Leitung endet pumpenseitig in einem ersten Anschluss dieser Kammer. Erfindungsgemäss weist die Kammer einen zweiten Anschluss zur Verbindung mit einem Milchsammelbehälter auf. Die zwei Anschlüsse stehen in der Kammer in fluidkommunizierender Verbindung miteinander. Die Leitung bildet während des Abpumpens eine Milchleitung zum Transport von in der Brusthaube abgepumpter Muttermilch zur Kammer. Die Milch wird anschliessend von der Kammer zum Milchsammelbehälter geleitet.

Vorteilhaft an diesem System ist nicht nur, dass die bereits abgepumpte Milch die Brusthaube wärmt, was für die Mutter angenehm ist. Es ist zudem nur eine einzige Leitung notwendig, welche sich insbesondere bei "hands-free" Ausführungen diskret in der Kleidung verstecken lässt.

Da das gesamte System von Milch geflutet ist und somit keine Vakuumleitung im klassischen Sinne mehr vorhanden ist, ist eine geringere Pumpleistung notwendig, um Muttermilch abzupumpen. Typische Werte für die Luftförderleistung liegen bei maximal 50 ml/min und für die Milchförderleistung bei max. 100 ml/min. Die Vakuumpumpe kann somit kleiner und leichter ausgestaltet werden, was wiederum für Aussenstehende weniger auffällig ist. Die Mutter kann diese Vakuumpumpe diskreter verwenden. Dank der geringeren Anforderung an die Pumpleistung ist die Vakuumpumpe im Gebrauch zudem leiser, was wiederum den Komfort und die Diskretion erhöht.

Da das gesamte System, d.h. das gesamte System bis auf den pumpaggregatseitigen oder antriebseitigen Bereich der Vakuumpumpe, mit Milch geflutet ist und keine oder nur sehr kleine luftgefüllten Toträume vorhanden sind, lässt sich das angelegte Vakuum leichter kontrollieren. Der an der Brusthaube anliegende Unterdruck entspricht auch eher dem in der Vakuumpumpe erzeugten Vakuum.

Diese Milchleitung lässt sich auf verschiedene Weisen verwirklichen. In einer bevorzugten Ausführungsform ist eine Scheidewand vorhanden ist, welche einen Antrieb der Vakuumpumpe und die Leitung voneinander trennt. Die Kammer wird dadurch von der Scheidewand in einen brusthaubenseitigen Bereich und einem antriebsseitigen Bereich unterteilt. Die zwei Bereiche sind vollständig voneinander getrennt und nur über die Membran miteinander verbunden. Somit ist gewährleistet, dass weder Milch in den antriebsseitigen Bereich der Vakuumpumpe gelangt, noch dass Verschmutzungen oder Luft des antriebsseitigen Bereichs in die milchführende Leitung und somit in die Brusthaube und den Milchsammelbehälter gelangt. Vorzugsweise ist diese Scheidewand eine Membran.

Diese Membran ist in einer bevorzugten Ausführungsform angetrieben und dient zur Förderung der abgepumpten Milch. Dadurch lässt sich die Milch unabhängig von der relativen Lage der Brusthaube, des Milchsammelbehälters und der Vakuumpumpe zueinander abpumpen. Die Mutter kann beispielsweise auch liegend abpumpen. Dies ist insbesondere bei einer "hands-free" Ausführung optimal, da sich die Mutter auch bücken und im Allgemeinen sehr frei bewegen kann.

Es lassen sich die unterschiedlichsten Vakuumpumpen mit der erfindungsgemässen vakuumübermittelnden und milchführenden Leitung verwenden. Vorzugsweise, jedoch nicht zwingend, wird jeweils eine einzige Membran sowohl für den Transport der Milch wie auch für die Medientrennung eingesetzt.

Vorzugsweise ist die Vakuumpumpe eine Membranpumpe, wobei die Kammer die vakuumerzeugende Pumpkammer der Vakuumpumpe ist und die Membran die für die Erzeugung des Vakuums verwendete Membran der Pumpkammer ist.

Die Brusthaube lässt sich auch direkt und ohne die oben beschriebene Leitung an ein Gehäuse einer Vakuumpumpe anschliessen. Trotzdem kann auf ein hydraulisches Pumpsystem gewechselt werden. Eine bevorzugte Vakuumpumpe zum Abpumpen von menschlicher Muttermilch mittels dieser Brusthaube weist einen Antrieb und eine vom Antrieb zyklisch antreibbare Membran auf, wobei die Membran in einer Kammer angeordnet ist, wobei die Membran die Kammer in einen antriebsseitigen Teil und einen brusthaubenseitigen Teil trennt und wobei der brusthaubenseitige Teil einen Vakuumanschluss zur Erstellung einer Verbindung zur Brusthaube aufweist. Erfindungsgemäss weist der brusthaubenseitige Teil der Kammer ferner einen Milchanschluss zur Erstellung einer Verbindung mit einem Milchsammelbehälter auf und der Vakuumanschluss und der Milchanschluss stehen über den brusthaubenseitigen Teil der Kammer in fluidkommunizierender Verbindung miteinander. Vorzugsweise ist diese Vakuumpumpe eine Membranpumpe und die Kammer ist die vakuumerzeugende Pumpkammer.

In einer bevorzugten Ausführungsform ist die Vakuumpumpe eine elektrische Membranpumpe. Die Membran der Membranpumpe bildet dabei vorzugsweise die als Milchantrieb und Scheidewand dienende oben genannte Membran. Der Antrieb der Membran dient vorzugweise gleichzeitig zur Erzeugung des Vakuums in der Pumpkammer und zur Förderung des Milchflusses. Dank der Dreifachfunktion der Membran lässt sich das Vakuum besser kontrollieren.

Vorzugsweise weist die Membran einen im Wesentlichen kreisförmigen Grundriss auf. Vorzugsweise ist die Membran in ihrem mittleren Bereich, vorzugsweise ihrer Mitte, angetrieben.

Es lassen sich auch andere Arten von Membranpumpen und alternative Pumpen ohne Membran können eingesetzt werden. Des weiteren können manuell betriebene Pumpen verwendet werden.

Die Kombination der drei Elemente
- vakuumübermittelnde und milchfuhrende Leitung
- Membran der Pumpkammer mit ihrer Dreifachfunktion
- die klein ausgebildete, Totvolumen vermeidende Brusthaube
führt zu einer Vorrichtung, welche äusserst klein und geräuscharm ausgebildet werden kann und ferner optimal für jegliche Art von Anwendung, insbesondere einer "hands-free" Anwendung, geeignet ist.

Weitere vorteilhafte Ausführungsformen und Varianten des Verfahrens sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: einen Längsschnitt durch eine erfindungsgemässe Brusthaube, angelegt an eine menschliche Brust mit grösserem Anpressdruck;
- Figur 2: einen Längsschnitt durch die erfindungsgemässe Brusthaube gemäss Figur 1, angelegt an eine menschliche Brust mit kleinerem Anpressdruck;
- Figur 3: einen Längsschnitt durch die Brusthaube gemäss Figur 1 mit einem Kopplungsteil in einer ersten Ausführungsform;
- Figur 4: einen Längsschnitt durch ein Kopplungsteil in einer zweiten Ausführungsform;
- Figur 5: einen Längsschnitt durch ein Kopplungsteil in einer dritten Ausführungsform;
- Figur 6: einen Längsschnitt durch das Kopplungsteil gemäss Figur 3;
- Figur 7: eine Explosionsdarstellung einer erfindungsgemässen Vorrichtung zum Abpumpen von menschlicher Muttermilch in einer ersten Ausführungsform, wobei eine Seitenwand der Vakuumpumpe nicht dargestellt ist;
- Figur 8: die Vorrichtung gemäss Figur 7 in gebrauchsmässigem Zusammenbau, wobei eine Seitenwand der Vakuumpumpe nicht dargestellt ist und
- Figur 9: eine erfindungsgemässe Vorrichtung in einer zweiten Ausführungsform, wobei eine Seitenwand der Vakuumpumpe nicht dargestellt ist.

Gleiche Teile sind in den Figuren mit denselben Bezugszeichen versehen.

### BESCHREIBUNG BEVORZUGTER AUSFÜHRUNGSFORMEN

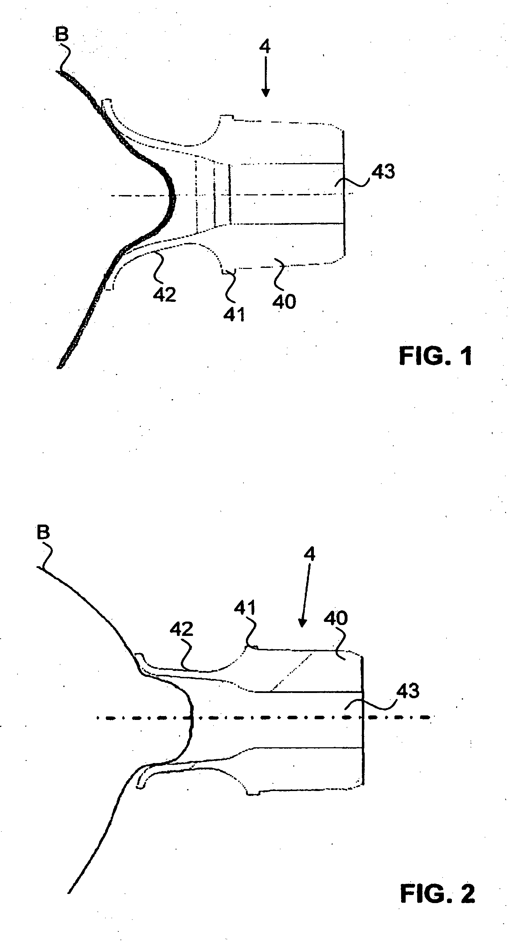

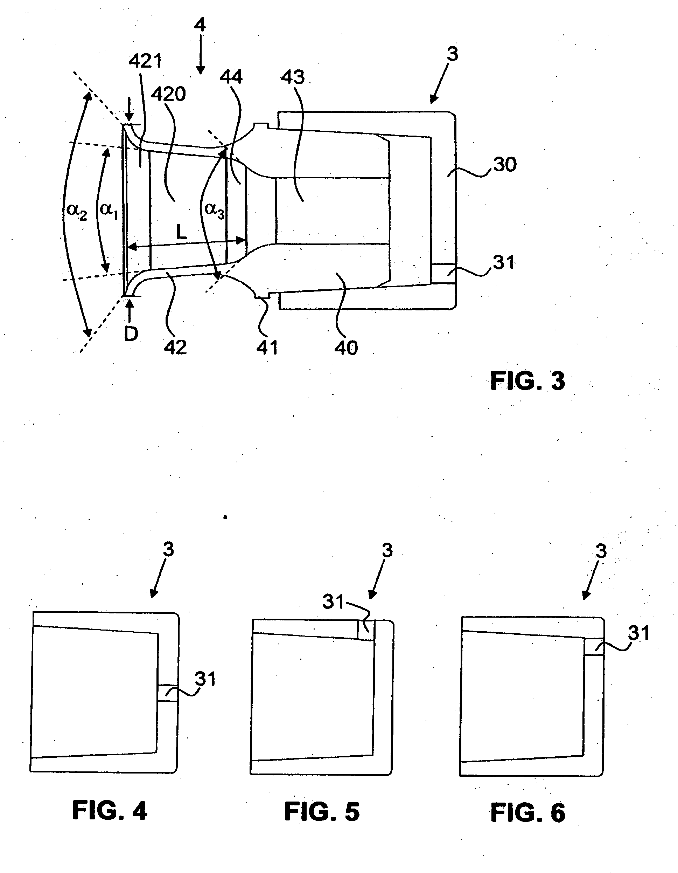

In den Figuren 1 bis 3 ist ein bevorzugtes Beispiel einer erfindungsgemässen Brusthaube 4 dargestellt. In Figur 3 ist sie im einen Brusthaubenset mit Kopplungsteil 3 sichtbar.

Figur 1 zeigt die erfindungsgemässe Brusthaube 4, wie sie an eine weibliche Brust B angelegt ist. Die Brusthaube 4 weist einen Stutzen 40, einen Trichter 42 und einen diese zwei verbindenden Übergangsbereich 44 auf. Vorzugsweise ist zwischen Stutzen 40 und Trichter 42 ein radial nach aussen vorstehender Flansch 41 vorhanden. Ein durchgehender Kanal 43 erstreckt sich durch die gesamte Brusthaube 4, so dass diese an zwei gegenüberliegenden Enden offen ausgebildet ist. Vorzugsweise ist die Brusthaube 4 rotationssymmetrisch ausgebildet. Vorzugsweise weist die Brusthaube 4 keinerlei weiteren Elemente auf, insbesondere keine Einsätze.

Vorzugsweise ist die Brusthaube 4 einstückig ausgebildet. Sie besteht üblicherweise aus Kunststoff, vorzugsweise aus Silikon.

Der Stutzen 40 ist massiv, d.h. relativ steif ausgebildet und dient zur Kopplung mit dem Kopplungsteil 3. Er weist eine um ein Vielfaches grössere Wandstärke auf als der Trichter 42. In diesem Beispiel ist der Stutzen 40 an seinem äusseren Umfang konisch ausgebildet, wobei er vorzugsweise rotationssymmetrisch ausgebildet ist. Die Konizität erleichtert das Einschieben in das Kopplungsteil 3 und erhöht zudem die Dichtheit der Verbindung mit dem Kopplungsteil 3. Die Dichtheit wird ferner erzielt, indem der Stutzen 40 materialbedingt im Kopplungsteil leicht zusammengedrückt wird. Hiefür ist der Aussendurchmesser des Stutzens 40 leicht grösser als der Innendurchmesser des Kopplungsteils 3. Typische äussere Durchmesser des Stutzens 40 betragen 8 mm bis 40 mm. Typische Längen betragen 5 mm bis 40 mm.

Der Trichter 42 dient zur Aufnahme der Mutterbrust. Er ist vorzugsweise sehr flexibel gestaltet. Er ist wesentlich flexibler und weicher ausgestaltet als der Stutzen 40. Dank seiner Flexibilität passt er sich in seiner Form der Form der Brust an. Er weist hier eine um ein Vielfaches kleinere Wandstärke auf als der Stutzen 40. Alternativ können Stutzen 40 und Trichter 42 auch dieselbe Wandstärke aufweisen, wobei in diesem Fall der Stutzen 40 vorzugsweise aus einem Material mit grösserer Shore-Härte gefertigt ist oder eine Verstärkung aufweist. Vorzugsweise weist der Trichter 42 eine Shore A Härte von circa 50 und der Stutzen 40 eine Shore A Härte von circa 70 auf.

In Figur 3 ist die Brusthaube 4 bei Nichtgebrauch dargestellt. Der Trichter 42 weist mindestens zwei Bereiche auf: einen Hauptbereich 420 und einen vorderen, brusthaubenseitigen Endbereich 421. Der Endbereich 421 bildet das brusthaubenseitige Ende der Brusthaube und endet somit frei.

Der Hauptbereich 420 weist bei Nichtgebrauch einen ersten Üffnungswinkel α₁ und der Endbereich einen zweiten Öffnungswinkel α₂ auf. Der erste Öffnungswinkel α₁ ist kleiner als der zweite Öffnungswinkel α₂. Zudem ist im Endbereich 421 die Wand vorzugsweise nach aussen umgebogen. Wie in den Figuren 1 und 2 erkennbar ist, lässt sich der erste Öffnungswinkel α₁ bei axialem Anpressdruck auf die Brust vergrössern, so dass sich der Endbereich 421 optimal an die Form der Brust anpassen kann.

Wie in den Figuren erkennbar ist, schliesst der Hauptbereich 420 unmittelbar an den Endbereich 421 an. Der Hauptbereich 420 schliesst am anderen Ende unmittelbar an den Übergangsbereich 44 an.

Im Übergangsbereich 44 ist ein weiterer dritter Öffnungswinkel α₃ vorhanden, welcher ebenfalls grösser als der erste Öffnungswinkel des Hauptbereichs 420 ist. Dieser dient dem Hauptbereich als Vorgabe beim Vergrössern des Winkels.

Die Winkel bei Nichtgebrauch betragen vorzugsweise für den ersten Öffnungswinkel α₁ ≤.5°, für den zweiten Öffnungswinkel α₂. 90° bis 160° und für den dritten Öffnungswinkel α₃ 60° bis 150°. Mindestens der erste Öffnungswinkel α₁ lässt sich bei Gebrauch vergrössern, vorzugsweise auf einen Winkel von bis zu 10°.

Der Trichter 42 weist eine Länge L von 10 mm bis 40 mm auf. Der Durchmesser D im vorderen Endbereich beträgt vorzugsweise 5 mm bis 40 mm, insbesondere 20 mm bis 40 mm. Dadurch ist er so klein, dass er die Brustwarze und keinen, höchstens einen Teil oder die gesamte Areola jedoch nicht die restliche Brust umfasst. Dies entspricht etwa dem Anteil der Brust, welche von einem Säugling in den Mund genommen wird. Der Trichter 42 ist im Bereich des Stutzens 40 kegelstumpfförmig gestaltet, wobei er sich zur Brust hin öffnet. Sein vorderer, brustseitiger Rand ist stärker nach aussen geneigt als der stutzenseitige Teil.

Da die Brusthaube 4 oder zumindest der Trichter 42 flexibel ausgestaltet ist, kann die Mutter durch Wahl des Anpressdrucks selber wählen, wie viel der Brust tatsächlich von der Brusthaube 4 umfasst wird. Der Anpressdruck ergibt sich aus dem axialen Druck auf den Trichter 42 und dem Gegendruck von der Mutterbrust. In Figur 1 ist der Anpressdruck relativ gross und der Trichter gespreizt, in Figur 2 ist der Druck kleiner und der Trichter 42 umschliesst lediglich die Brustwarze. Durch Wahl des Anpressdrucks lässt sich auch die Dichtheit der Anlage an der Brust einstellen und somit das Abpumpen für die Mutter so bequem wie möglich einstellen.

Die Brusthaube 4 ist, wie dies in Figur 3 erkennbar ist, in das Kopplungsteil 3 eingesteckt.

Das Kopplungsteil 3 ist vorzugsweise ebenfalls klein, aber möglichst steif ausgestaltet. Es ist in seinem äusseren Umfang vorzugsweise zylinderförmig ausgebildet und in seinem inneren Umfang kegelstumpfförmig bzw. konisch. Es weist einen u-förmigen Querschnitt auf, ist also an einem Ende offen und am gegenüberliegenden Ende geschlossen ausgebildet. Somit ist ein Sackloch vorhanden, in welches der Stutzen 40 der Brusthaube 4 bis zum Anschlag 41 eingeschoben werden kann. In Figur 3 ist die Brusthaube 4 noch nicht vollständig eingeschoben. Wie jedoch erkennbar ist, ist im vollständig eingeschobenen Zustand zwischen stirnseitigern Ende des Stutzens 40 und der Rückwand des Kopplungsteils 3 ein Spalt vorhanden, welcher einen Fluiddurchlass vom Kanal 43 zu einer Anschlussöffnung 31 im Kopplungsteil 3 bildet.

Diese Anschlussöffnung 31 dient zum Anschluss einer Vakuum- und/oder Milchleitung. Verlaufen Vakuumleitung und Milchleitung getrennt voneinander, so sind im Kopplungsteil 3 zwei Anschlussöffnungen vorhanden. Diese Anschlussöffnungen können mit Rückschlagventilen versehen sein. Die Vakuumleitung führt zur Vakuumpumpe, die Milchleitung zum Milchsammelbehälter.

Die Leitungen können einfach in die Öffnungen eingesteckt sein. Sie können aber auch fest mit dem Kopplungsteil 3 verbunden sein oder es können Ein- oder Aufsteckmittel, beispielsweise Stutzen, zur Verbindung mit den Leitungen vorhanden sein.

Die mindestens eine Anschlussöffnung 31 kann an verschiedenen Orten angebracht sein. In Figur 3 ist sie im oberen Bereich in der Rückwand des Kopplungsteils 3 angeordnet. In Figur 4 ist sie mittig in der Rückwand angeordnet. In Figur 5 ist sie im Mantel, jedoch im hinteren, der Rückwand nahen Bereich angeordnet. Vorzugsweise ebenfalls im oberen Bereich. Figur 6 zeigt nochmals die Situation gemäss Figur 3, jedoch ohne eingesteckte Brusthaube 4.

Ist die Anschlussöffnung 31 insbesondere für die Milchleitung oben angeordnet, so wird die im Kopplungsteil 3 vorhandene Restluft zusammen mit der Milch abgesaugt und das Totvolumen wird nochmals reduziert. Brusthaube 4 und Kopplungsteil 3 weisen im Gebrauchszustand keine Luftkammern mehr auf. Ihre Freiräume, sofern noch vorhanden, sind mit Milch gefüllt. Damit die Anschlussöffnung 31 bei Gebrauch tatsächlich nach oben gerichtet ist, kann beispielsweise eine entsprechende Markierung auf dem Kopplungsteil 3 angegeben sein.

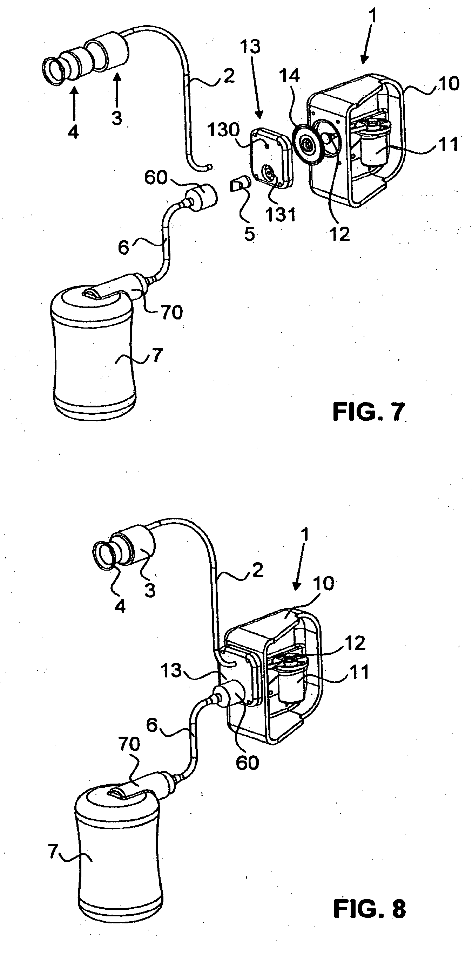

Diese Brusthaube lässt sich mit allen Arten von Brustpumpen verwenden. Vorteilhaft ist sie jedoch bei Verwendung mit den in den Figuren 7 bis 9 dargestellten Vorrichtungen zum Abpumpen von menschlicher Muttermilch.

In den Figuren 7 und 8 ist eine erste Ausführungsform einer derartigen Vorrichtung dargestellt. Sie weist eine Vakuumpumpe 1, eine erste Leitung 2, das Kopplungsteil 3, die Brusthaube 4, ein Rückschlagventil 5, eine zweite Leitung 6 und einen Milchsammelbehälter 7 auf.

Die Brusthaube 4 ist über das Kopplungsteil 3 und der ersten flexiblen Leitung 2 mit der Vakuumpumpe 1 verbunden. Von der Vakuumpumpe 1 führt die zweite flexible Leitung 6 zum Milchsammelbehälter 7, wobei diese Verbindung mit dem Rückschlagventil 5 versehen ist. Die zwei flexiblen Leitungen 2, 6 sind vorzugsweise Schläuche, insbesondere aus Silikon.

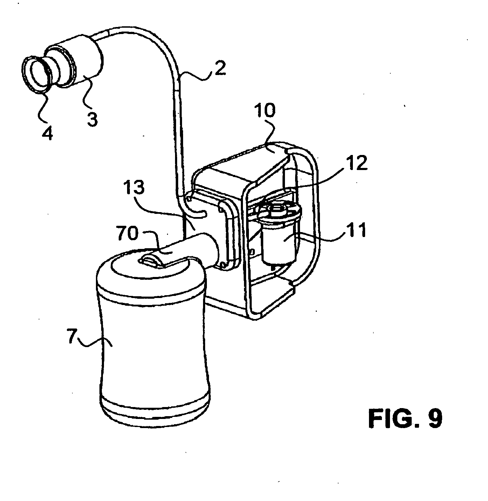

Wie in Figur 9 dargestellt ist, kann alternativ der Milchsammelbehälter 7 auch direkt an der Vakuumpumpe 1 befestigt sein. Hierfür ist vorzugsweise ein geeignet geformter Verbindungsstutzen 70 am Milchsammelbehälter 7 vorhanden, welcher mit einem Gehäuse 10 der Vakuumpumpe lösbar verbindbar ist.

Die Vakuumpumpe 1 weist das genannte Gehäuse 10 auf, wobei in den Zeichnungen eine Seitenwand des Gehäuses 10 nicht dargestellt ist. Dadurch lässt sich das Innere des Gehäuses 10 erkennen.

Im Gehäuse 10 ist ein Elektromotor 11 vorhanden. Er kann netzwerkbetrieben und/oder batteriebetrieben sein. Eine Kraftübertragungseinheit 12, hier eine mit dem Motor verbundene Pleuelstange, überträgt die Drehbewegung des Motors in eine lineare Bewegung. Die Pleuelstange 12 ist an ihrem zweiten Ende mit einer Membran 14 verbunden. Die Membran 14 ist in einer Ausnehmung des Gehäuses 10, welche ein Teil einer Pumpkammer bildet, angeordnet. Ein mit dem Gehäuse 10 lösbar verbindbarer Deckel 13 hält die Membran 14 in ihrer Position fest.

Anstelle dieses Antriebs lassen sich auch andere Arten von Antrieben, insbesondere auch manuelle Antriebe, einsetzen.

Der Deckel 13 ist vorzugsweise am Gehäuse 10 angeschraubt. Andere Verbindungsarten, sind ebenfalls möglich. Der Deckel 13 weist ebenfalls eine Ausnehmung auf, so dass er einen zweiten Teil der Pumpkammer bildet. Die zwei Teile der Pumpkammer sind durch die Membran 14 voneinander getrennt. Der Deckel 13 kann einstückig oder mehrstückig ausgebildet sein.

Die Wirkungsweise der Membranpumpe wird hier nicht im Detail beschrieben, da sie hinlänglich bekannt ist. Mittels eines Antriebs, hier mittels Motor 11 und Pleuelstange 12, wird die Membran zyklisch hin- und her bewegt, so dass im brusthaubenseitigen oder deckelseitigen Teil der Pumpkammer ein Unterdruck erzeugt wird. Anstelle des hier dargestellten Antriebs lassen sich auch andere Arten von Antrieben verwenden, welche geeignet sind, die Membran 14 zyklisch zu bewegen. Die für die Betreibung der Pumpe notwendige Elektronik sowie die Bedienungselemente sind hier nicht dargestellt. Es können bekannte Mittel verwendet werden. Die Pumpe lässt sich mit zeitlich gleichbleibendem Zyklus betreiben oder die Saugkurve kann, wie dies im Stand der Technik bekannt ist, in ihrer Form, Frequenz und Intensität, dem Saugvechalten des Säuglings und/oder den Bedürfnissen der Mutter angepasst sein.

Im Deckel 13, d.h. in der Ventilplatte, ist eine erste Auslassöffnung 130 vorhanden, welche die Umwelt mit dem deckelseitigen Teil der Pumpkammer verbindet. Diese Auslassöffnung 130 dient als erster Anschluss für die erste Leitung 2. Eine zweite Auslassöffnung 131, welche ebenfalls den deckel- bzw. brustseitigen Teil der Pumpkammer mit der Umgebung verbindet, ist als zweiter Anschluss ausgebildet. Dieser zweite Anschluss ist mit dem Rückschlagventil 5 versehen. Hier wird ein Schnabelventil verwendet, welches auf einen Stutzen aufgesteckt ist. Andere Ventilarten sind jedoch auch einsetzbar.

Wird die Vorrichtung nun verwendet, so wird die Brusthaube 4 auf die Mutterbrust aufgesetzt, so dass sie mindestens die Brustwarze umfasst. Vorzugsweise ist zusätzlich maximal die Areola von der Brusthaube 4 umfasst. Die Vakuumpumpe 1 wird eingeschaltet und in bekannter Weise betrieben. Das in der Pumpkammer erzeugte Vakuum evakuiert die erste Leitung 2, so dass in der Brusthaube 4 ein Unterdruck vorliegt. Dadurch wird Milch aus der Mutterbrust abgepumpt und gelangt durch die Brusthaube 4 und das Kopplungsteil 3 in die erste Leitung 2. Die Milch fliesst durch den ersten Anschluss 130 in den deckelseitigen Teil der Pumpkammer. Die abgepumpte Milch verlässt die Pumpkammer durch den zweiten Anschluss 131 und das Rückschlagventil 5 und gelangt über die zweite Leitung 6 (siehe Figur 8) bzw. je nach Ausführungsform auch direkt in den Milchsammelbehälter (siehe Figur 9). Es ist somit keine separate Leitung für den Milchtransport vorhanden. Die erste Leitung 2 dient gleichzeitig als Saugleitung und als Milchtransportleitung. Die Vorrichtung wechselt somit nach anfanglichem pneumatischen Pumpen in ein hydraulisches Pumpen. Dies ist eine weitere Annäherung an das natürliche Saugen von Säuglingen.

Die Membran 14 in der Pumpkammer weist drei Funktionen auf. Erstens bildet sie die Membran der Membranvakuumpumpe und erzeugt so das Vakuum in der Pumpkammer. Zweitens dient sie als Scheidewand zwischen der Luft im pumpenseitigen Teil der Pumpkammer und der Milch im deckelseitigen Teil der Pumpkammer. Sie dient somit als Medientrennung. Dadurch verhindert sie, dass Milch in das Pumpaggregat gelangen kann. Sie verhindert aber auch, dass Verschmutzungen des Pumpaggregats in die erste und zweite Leitung 2, 6 gelangen können. Drittens führt ihre zyklische Bewegung innerhalb der Pumpkammer dazu, dass sie die Milch fördert und transportiert. Dank dieser dritten Funktion der Membran 14 können während des Abpumpens Milchsammelbehälter 7, Brusthaube 4 und Vakuumpumpe 1 in voneinander unabhängigen Lagen angeordnet sein. Beispielsweise kann sich der Milchsammelbehälter 7 oberhalb der Vakuumpumpe 1 und/oder der Brusthaube 4 befinden. Auch die Vakuumpumpe 1 kann oberhalb des Milchsammelbehälters 7 und/oder der Brusthaube 4 sein. Dies ermöglicht der Mutter auch liegend abzupumpen oder, wenn sie sitzt, den Milchsammelbehälter 7 und die Vakuumpumpe 1 ausser Reichweite von Kleinkindern auf ein Regal oder eine andere erhöhte Plattform zu stellen.

Vorzugsweise wird in den Beispielen gemäss den Figuren 7 bis 9 ein Unterdruck von 0 bis 300 mmHg erzeugt. Die Pumpfrequenz ist vorzugsweise zwischen 5 und 120 Zyklen/Minute.

Das Rückschlagventil 5 öffnet sich vorzugsweise erst bei genügendem Druck, d.h. wenn die Pumpkammer ausreichend mit Milch gefüllt ist. Dadurch lässt sich das Totvolumen, welches evakuiert werden muss, minimal halten.

Die erfindungsgemässe Brusthaube ist für die Mutter angenehm zu tragen und reduziert das luft- oder milchgefüllte Totvolumen beim Abpumpen von Muttermilch auf ein Mimimum.

**BEZUGSZEICHENLISTE**

| | | | |
|---|---|---|---|
| 1 | Vakuumpumpe | 421 | Endbereich |
| 10 | Gehäuse | 43 | Kanal |
| 11 | Motor | 44 | Übergangsbereich |
| 12 | Kraftübertragungseinheit | | |
| 13 | Deckel | 5 | Rückschlagventil |
| 130 | erster Anschluss | | |
| 131 | zweiter Anschluss | 6 | zweite Leitung |
| 133, 133' | Ausnehmung | 60 | Ventilkappe |
| 14 | Membran | | |
| | | 7 | Milchsammelbehälter |
| 2 | erste Leitung | 70 | Verbindungsstutzen |
| | | | |
| 3 | Kopplungsteil | B | Brust |
| 30 | Grundkörper | D | Durchmesser des Endbereichs des Trichters |
| 31 | Anschlussöffnung | | |
| | | L | Länge des Trichters |
| 4 | Brusthaube | α₁ | erster Öffnungswinkel |
| 40 | Stutzen | α₂ | zweiter Öffnungswinkel |
| 41 | Anschlag | α₃ | dritter Öffnungswinkel |
| 42 | Trichter | | |
| 420 | Hauptbereich | | |

## Patentansprüche

1. Brusthaube (4) zur Verwendung in einer Vorrichtung zum Abpumpen von menschlicher Muttermilch, wobei die Brusthaube (4) einen rohrförmigen Stutzen (40) und einen daran einstückig angeformten Trichter (42) zur Auflage an eine Mutterbrust (B) aufweist, wobei der Trichter (42) sich zu seiner freien, dem Stutzen (40) abgewandten Seite hin erweitert und wobei ein Kanal (43) vorhanden ist, welcher sich von einem brustseitigen Ende des Trichters (42) durchgehend bis zu einem, diesem Ende gegenüberliegenden, pumpenseitigen Ende des Stutzens (40) erstreckt und welcher zum Anlegen eines Vakuums an die Mutterbrust und zum Abfliessen der abgepumpten Muttermilch dient, **dadurch gekennzeichnet, dass** der Trichter (42) flexibler ausgebildet ist als der Stutzen (40), dass der Trichter (42) einen sich über einen wesentlichen Teil seiner Länge erstreckenden Hauptbereich (420) mit einem ersten Öffnungswinkel (α₁) des Kanals (43) und einen brustseitigen Endbereich (421) mit einem zweiten Öffnungswinkel (α₂) des Kanals (43) aufweist, wobei der erste Öffnungswinkel (α₁) bei Nichtgebrauch kleiner ist als der zweite Öffnungswinkel (α₂) und dass im Gebrauchszustand mindestens der erste Öffnungswinkel (α₁) durch axialen Druck auf die Brusthaube (4) vergrösserbar ist.

2. Brusthauben nach Anspruch 1, wobei der Trichter (42) einen brustseitigen Durchmesser (D) von 5 mm bis 40 mm und eine Länge (L) von 10 mm bis 40 mm aufweist, so dass im Gebrauchszustand die Brustwarze und maximal die Areola von der Brusthaube (4) umfasst ist.

3. Brusthaube nach einem der Ansprüche 1 oder 2, wobei zwischen Stutzen (40) und Trichter (42) ein Übergangsbereich (44) vorhanden ist mit einem dritten Öffnungswinkel (α₃) des Kanals (43), wobei der dritte Öffnungswinkel (α₃) bei Nichtgebrauch grösser ist als der erste Öffnungswinkel (α₁).

4. Brusthaube nach Anspruch 3, wobei der Hauptbereich (420) unmittelbar an den Übergangsbereich (44) anschliesst.

5. Brusthaube nach einem der Ansprüche 1 oder 2, wobei der Endbereich (421) unmittelbar an den Hauptbereich (420) anschliesst

6. Brusthaube nach einem der Ansprüche 1 bis 5, wobei der Stutzen (40) eine um ein Vielfaches grössere Wandstärke aufweist als der Trichter (42).

7. Brusthaube nach einem der Ansprüche 1 bis 6, wobei im Übergangsbereich (44) zwischen Stutzen (40) und Trichter (42) ein äusserer Anschlag (41) vorhanden ist, welcher dem äusseren Umfang des Stutzens (40) vorsteht.

8. Brusthaube nach einem der Ansprüche 1 bis 7, wobei der Stutzen (40) in seinem äusseren Umfang sich konisch zum Trichter (42) hin erweiternd ausgebildet ist.

9. Brusthaube nach einem der Ansprüche 1 bis 8, wobei sie aus Silikon gefertigt ist.

10. Brusthaube nach einem der Ansprüche 1 bis 9, wobei der Trichter (42) eine Shore A Härte von circa 50 und der Stutzen (40) eine Shore A Härte von circa 70 aufweist.

11. Brusthaubenset mit einer Brusthaube (4) nach einem der Ansprüche 1 bis 10 und einem Kopplungsteil (3) zur dichtenden Aufnahme des Stutzens (40) der Brusthaube (4), **dadurch gekennzeichnet, dass** das Kopplungsteil (3) zylinderförmig ausgebildet ist und auf einer Seite mit einem Boden verschlossen ist, so dass ein Sackloch zur Aufnahme des Stutzens (40) gebildet ist und dass mindestens eine Anschlussöffnung (31) vorhanden ist, welche in fluidkommunizierender Verbindung mit dem Kanal (43) der Brusthaube (4) steht.

12. Brusthaubenset nach Anspruch 11, wobei im zusammengebauten Zustand ein brusthaubenfernes, stirnseitiges Ende des Stutzens (40) beabstandet zum Boden des Kopplungsteils (3) endet.

13. Brusthaubenset nach einem der Ansprüche 11 oder 12, wobei die mindestens eine Anschlussöffnung (31) azentrisch im Kopplungsteil (3) angeordnet ist.

14. Brusthaubenset nach Anspruch 13, wobei die mindestens eine Anschlussöffnung (31) in einem oberen Bereich des Kopplungsteils (3) angeordnet ist und wobei das Kopplungsteil (3) eine Markierung aufweist, welche "oben" definiert.

15. Brusthaübenset nach einem der Ansprüche 11 bis 14, wobei das Sackloch des Kopplungsteils (3) sich im Durchmesser zum Boden hin verjüngt.

16. Brusthaubenset nach einem der Ansprüche 11 bis 15, wobei das Kopplungsteil (3) steif ausgebildet ist.

## Claims

1. A breast shield (4) for use in a device for expressing human breast milk, wherein the breast shield (4) has a tubular connector (40) and a funnel (42) that is integrally formed thereon and is intended for resting on a mother's breast (B), wherein the funnel (42) widens toward the free side thereof which faces away from the connector (40), and wherein there is a passage (43) which extends continuously from a breast-side end of the funnel (42) as far as a pump-side end, facing said breast-side end, of the connector (40) and which serves to apply a vacuum to the mother's breast and for the flowing away of the expressed breast milk, **characterized in that** the funnel (42) is of more flexible design than the connector (40), **in that** the funnel (42) has a main region (420) extending over a substantial part of the length thereof with a first opening angle (α₁) of the passage (43), and a breast-side end region (421) with a second opening angle (α₂) of the passage (43), wherein, when not in use, the first opening angle (α₁) is smaller than the second opening angle (α₂), and **in that**, in the use state, at least the first opening angle (α₁) can be enlarged by axial pressure on the breast shield (4).

2. The breast shield as claimed in claim 1, wherein the funnel (42) has a breast-side diameter (D) of 5 mm to 40mm and a length (L) of 10 mm to 40 mm, and therefore, in the use state, the nipple, and at maximum the areola, is surrounded by the breast shield (4).

3. The breast shield as claimed in either of claims 1 or 2, wherein there is a transition region (44) between the connector (40) and funnel (42), with a third opening angle (α₃) of the passage (43), wherein, when not in use, the third opening angle (α₃) is greater than the first opening angle (α₁).

4. The breast shield as claimed in claim 3, wherein the main region (420) directly adjoins the transition region (44).

5. The breast shield as claimed in claim 1 or 2, wherein the end region (421) directly adjoins the main region (420).

6. The breast shield as claimed in one of claims 1 to 5, wherein the connector (40) has a much greater wall thickness than the wall thickness of the funnel (42).

7. The breast shield as claimed in one of claims 1 to 6, wherein there is an outer stop (41), which protrudes over the outer circumference of the connector (40), in the transition region (44) between the connector (40) and funnel (42).

8. The breast shield as claimed in one of claims 1 to 7, wherein the outer circumference of the connector (40) is designed such that it widens conically toward the funnel (42).

9. The breast shield as claimed in one of claims 1 to 8, wherein it is manufactured from silicone.

10. The breast shield as claimed in one of claims 1 to 9, wherein the funnel (42) has a Shore-A hardness of about 50 and the connector (40) has a Shore-A hardness of about 70.

11. A breast shield set with a breast shield (4) as claimed in one of claims 1 to 10 and a coupling part (3) for receiving the connector (40) of the breast shield (4) in a sealing manner, wherein the coupling part (3) is of cylindrical design and is closed on one side by a base such that a blind hole for receiving the connector (40) is formed, and wherein there is at least one port opening (31) which is connected in terms of fluid communication to the passage (43) of the breast shield (4).

12. The breast shield set as claimed in claim 11, wherein, in the assembled state, a breast-shield-remote front end of the connector (40) ends at a distance from the base of the coupling part (3).

13. The breast shield set as claimed in either of claims 11 and 12, wherein the at least one port opening (31) is arranged eccentrically in the coupling part (3).

14. The breast shield set as claimed in claim 13, wherein the at least one port opening (31) is arranged in an upper region of the coupling part (3), and wherein the coupling part (3) has a marking which defines "at the top".

15. The breast shield set as claimed in one of claims 11 to 14, wherein the blind hole of the coupling part (3) tapers in diameter toward the base.

16. The breast shield set as claimed in one of claims 11 to 15, wherein the coupling part (3) is of stiff design.

## Revendications

1. Téterelle (4) pour l'utilisation dans un dispositif pour pomper du lait maternel humain, la téterelle (4) présentant un raccord tubulaire (40) et un entonnoir (42) formé d'une seule pièce sur celui-ci, destiné à s'appliquer contre un sein maternel (B), l'entonnoir (42) s'élargissant vers son côté libre opposé au raccord (40) et un canal (43) étant prévu, lequel s'étend depuis une extrémité de l'entonnoir (42) du côté du sein en continu jusqu'à une extrémité du raccord (40) du côté de la pompe, opposée à cette extrémité, et lequel sert à appliquer un vide au niveau du sein maternel et à évacuer le lait maternel pompé, **caractérisée en ce que** l'entonnoir (42) est réalisée sous forme plus flexible que le raccord (40), **en ce que** l'entonnoir (42) présente une région principale (420) s'étendant sur une majeure partie de sa longueur, avec un premier angle d'ouverture (α₁) du canal (43) et une région d'extrémité (421) du côté du sein, avec un deuxième angle d'ouverture (α₂) du canal (43), le premier angle d'ouverture (α₁), dans l'état de non utilisation, étant inférieur au deuxième angle d'ouverture (α₂), et **en ce que**, dans l'état d'utilisation, au moins le premier angle d'ouverture (α₁) peut être augmenté par pression axiale sur la téterelle (4).

2. Téterelle selon la revendication 1, l'entonnoir (42) présentant un diamètre (D) du côté du sein de 5 mm à 40 mm et une longueur (L) de 10 mm à 40 mm, de sorte que dans l'état d'utilisation, le mamelon et au maximum l'aréole soient entourés par la téterelle (4).

3. Téterelle selon l'une quelconque des revendications 1 ou 2, une région de transition (44) étant prévue entre le raccord (40) et l'entonnoir (42), avec un troisième angle d'ouverture (α₃) du canal (43), le troisième angle d'ouverture (OE₃) en cas de non utilisation, étant supérieur au premier angle d'ouverture (α₁).

4. Téterelle selon la revendication 3, la région principale (420) se raccordant directement à la région de transition (44).

5. Téterelle selon l'une quelconque des revendications 1 ou 2, la région d'extrémité (421) se raccordant directement à la région principale (420).

6. Téterelle selon l'une quelconque des revendications 1 à 5, le raccord (40) présentant une épaisseur de paroi plusieurs fois supérieure à celle de l'entonnoir (42).

7. Téterelle selon l'une quelconque des revendications 1 à 6, une butée extérieure (41) étant prévue dans la région de transition (44) entre le raccord (40) et l'entonnoir (42), laquelle fait saillie depuis la périphérie extérieure du raccord (40).

8. Téterelle selon l'une quelconque des revendications 1 à 7, le raccord (40) étant réalisé de manière à s'élargir coniquement vers l'entonnoir (42) dans sa périphérie extérieure.

9. Téterelle selon l'une quelconque des revendications 1 à 8, laquelle est fabriquée en silicone.

10. Téterelle selon l'une quelconque des revendications 1 à 9, l'entonnoir (42) présentant une dureté Shore A d'environ 50 et le raccord (40) présentant une dureté Shore A d'environ 70.

11. Ensemble de téterelle comprenant une téterelle (4) selon l'une quelconque des revendications 1 à 10 et une partie d'accouplement (3) pour recevoir hermétiquement le raccord (40) de la téterelle (4), **caractérisé en ce que** la partie d'accouplement (3) est réalisée sous forme cylindrique et est fermée d'un côté par un fond, de telle sorte qu'un trou borgne soit formé pour recevoir le raccord (40), et **en ce qu'**au moins une ouverture de raccordement (31) est prévue, laquelle est en liaison fluidique avec le canal (43) de la téterelle (4).

12. Ensemble de téterelle selon la revendication 11, une extrémité frontale du raccord (40), éloignée de la téterelle, se terminant, dans l'état assemblé, à distance du fond de la partie d'accouplement (3).

13. Ensemble de téterelle selon l'une quelconque des revendications 11 ou 12, l'au moins une ouverture de raccordement (31) étant disposée de manière décentrée dans la partie d'accouplement (3).

14. Ensemble de téterelle selon la revendication 13, l'au moins une ouverture de raccordement (31) étant disposée dans une région supérieure de la partie d'accouplement (3) et la partie d'accouplement (3) présentant un marquage qui définit le "haut".

15. Ensemble de téterelle selon l'une quelconque des revendications 11 à 14, dans lequel le trou borgne de la partie d'accouplement (3) a un diamètre qui se rétrécit en direction du fond.

16. Ensemble de téterelle selon l'une quelconque des revendications 11 à 15, la partie d'accouplement (3) étant réalisée sous forme rigide.
